# EUROPÄISCHE PATENTANMELDUNG

(11) **EP 3 085 399 A1**
(43) Veröffentlichungstag der Anmeldung: **26.10.2016**
(21) Anmeldenummer: 15164986.0
(22) Anmeldetag: 24.04.2015
(51) Int. Cl.: A61M 1/16

(54) **Dialysetrockenkonzentrat-Kartusche**

(71) Anmelder: D_MED Consulting AG, 47269 Duisburg (DE)
(72) Erfinder: Breuch, Gert, 53844 Troisdorf (DE); Biermann, Frank, 22455 Hamburg (DE); Breuch, Julius, 20457 Hamburg (DE); Holzschuh, Robert, 22301 Hamburg (DE)
(74) Vertreter: Patentanwälte ter Smitten Eberlein Rütten Partnerschaftsgesellschaft

(57) **Zusammenfassung**

Die Erfindung bezieht sich auf eine Dialysetrockenkonzentrat-Kartusche (10) mit einem in einer Lösungsflüssigkeit lösbaren Trockenkonzentrat (44) zur Herstellung einer Dialyseflüssigkeit durch Mischen des Trockenkonzentrats (44) mit einer Lösungsflüssigkeit. Die Kartusche weist einen steifen Verbindungskörper (20) mit einem Kartuschen-Einlass (18) an einem Längsende (21) und einem koaxialen Kartuschen-Auslass (19) an dem anderen Längsende (31) auf. An dem Verbindungskörper ist mindestens eine Zulauföffnung (28) vorgesehen, durch die die Lösungsflüssigkeit in einen Mischraum (54) fließt, der den Verbindungskörper (20) teilweise umgibt. Ferner ist an dem Verbindungskörper mindestens eine Ablauföffnung (32) vorgesehen, durch die die Lösungsflüssigkeit aus dem Mischraum (54) zu dem Auslass (19) abfließt. Die Kartusche weist einen separaten flexiblen Mischraumbalg (40) auf, der den Mischraum (54) zwischen der Zulauföffnung (28) und der Ablauföffnung (32) fluidisch abschließt.

## Beschreibung

Die Erfindung bezieht sich auf eine Dialysetrockenkonzentrat-Kartusche mit einem in einer Lösungsflüssigkeit lösbaren Trockenkonzentrat, wobei die Dialysetrockenkonzentrat-Kartusche der Herstellung einer Dialyseflüssigkeit durch Mischen des Trockenkonzentrats mit einer Lösungsflüssigkeit, beispielsweise mit Dialysewasser, dient.

Eine Trockenkonzentrat-Kartusche zur Herstellung einer Desinfektions-Flüssigkeit, in einem Dialysegerät ist aus EP 0 458 041 A1 bekannt. Die Kartusche wird im Wesentlichen von einem steifen Kartuschengehäuse gebildet, in dem das Trockenkonzentrat gelagert ist, und weist einen Kartuschen-Einlass an einem oberen Längsende und einem koaxialen Kartuschen-Auslass an dem unteren Längsende auf. Zur Herstellung der Desinfektions-Flüssigkeit oder einer anderen Flüssigkeit wird die Kartusche zunächst in eine geräteseitigen Einspannvorrichtung vertikal eingespannt. Die axiale Länge der Kartusche ist exakt auf die korrespondierenden Anschlusselemente der Einspannvorrichtung abgestimmt, so dass eine Kartusche mit einer abweichenden axialen Länge nicht eingespannt werden kann.

Das Volumen des Kartuschengehäuses ist auf die eingelagerte Trockenkonzentrat-Menge und das erforderliche Mischraum-Volumen abgestimmt, das für eine betriebssichere Auflösung des Trockenkonzentrats in der Lösungsflüssigkeit erforderlich ist.

Die Trockenkonzentrat-Kartusche ist in der Regel ein sogenannter Einmalartikel, wird also nach einmaliger Verwendung entsorgt. Wegen ihres relativ großen Volumens verursacht die Trockenkonzentrat-Kartusche auch ein entsprechend großes Abfallvolumen.

Aufgabe der Erfindung ist es, eine Dialysetrockenkonzentrat-Kartusche mit einem geringen Abfallvolumen zu schaffen.

Diese Aufgabe wird erfindungsgemäß mit einer Dialysetrockenkonzentrat-Kartusche mit den Merkmalen des Anspruchs 1 gelöst.

Die erfindungsgemäße Dialysetrockenkonzentrat-Kartusche weist einen steifen Verbindungskörper mit einem Kartuschen-Einlass an dem einem Längsende und einem hierzu koaxial angeordneten Kartuschen-Auslass an dem anderen Verbindungskörper-Längsende auf. Der steife Verbindungskörper definiert den exakten axialen Abstand des Kartuschen-Einlasses und des Kartuschen- Auslasses sowie die Koaxialität des Einlasses und Auslasses zueinander. Auf diese Weise ist sichergestellt, dass die Kartusche exakt auf die korrespondierende Einspannvorrichtung eines Dialysegerätes abgestimmt ist.

Der Verbindungskörper weist stromabwärts des Einlasses eine Zulauföffnung auf, durch die die Lösungsflüssigkeit in einen Mischraum einfließen kann, der den Verbindungskörper teilweise umgibt, und besonders bevorzugt zirkulär vollständig umgibt. Der Mischraum erstreckt sich jedoch nicht notwendigerweise über die gesamte axiale Länge des Verbindungskörpers. Ferner weist der Verbindungskörper eine Ablauföffnung auf, durch den die Lösungsflüssigkeit aus dem Mischraum zu dem Auslass fließt. Der Mischraum wird von einem separaten flexiblen Mischraumbalg abgeschlossen, wobei der Mischraum fluidisch zwischen der Zulauföffnung und der Ablauföffnung angeordnet ist. Der Mischraum ist der Raum, in dem das Trockenkonzentrat mit der Lösungsflüssigkeit zusammengebracht und vermischt wird, um von dem Mischraum aus als Dialyseflüssigkeit durch die Ablauföffnung und den Ablauf abzufließen.

Wenn die Kartusche leer ist bzw. nicht mehr benötigt wird, lässt sich der Mischraum durch entsprechende Verformung des Mischraumbalgs verkleinern, so dass der Mischraum anschließend stark verkleinert ist oder nicht mehr vorhanden ist. Beispielsweise kann der Mischraumbalg auf das Volumen des Verbindungskörpers zusammengedrückt werden. Auf diese Weise wird das Abfallvolumen der Kartusche erheblich verringert, ohne dass hierdurch die Funktionalität der Dialysetrockenkonzentrat-Kartusche beschränkt würde bzw. der Mischraum während des Mischbetriebes verkleinert wäre.

Vorzugsweise wird der Verbindungskörper von einem steifen Rohrkörper gebildet. Das eine Längsende des Rohrkörpers bildet den Einlass, durch den das Lösungsmittel in die Kartusche einfließt, und das andere Längsende des Rohrkörpers bildet den Kartuschen-Auslass, durch den die in Mischraum generierte Dialyseflüssigkeit die Kartusche verlässt. Ein Rohrkörper ist einfach und preiswert zu beschaffen und zu bearbeiten, so dass die Beschaffungs- und Herstellungskosten für den Verbindungskörper gering sind.

Vorzugsweise ist in dem Rohrkörper zwischen dem Einlass und dem Auslass eine Trennwand angeordnet. Hierdurch wird die durch den Kartuschen-Einlass an dem einen offenen Längsende des Rohrkörpers einströmende Lösungsflüssigkeit gezwungen, durch die Zulauföffnung, die in der Rohrwand des Rohrkörper angeordnet ist, aus dem Rohrkörper-Hohlraum radial nach außen in den Mischraum zu fließen. Aus dem Mischraum fließt die Flüssigkeit durch die Ablauföffnung in der Rohrwand des Rohrkörpers radial nach innen in den Hohlraum des Rohrkörpers wieder ein, von wo aus die Flüssigkeit axial durch den Rohrkörper zu dem Kartuschen-Auslass strömt. Die Trennwand bildet also eine fluiddichte Trennung des Rohrkörper-Zulaufabschnitts von dem Rohrkörper-Ablaufabschnitt.

Gemäß einer bevorzugten Ausgestaltung der Erfindung sind stromaufwärts der Trennwand mehrere Zulauföffnungen und stromabwärts der Trennwand mehrere Ablauföffnungen in der Rohrwand des Rohrkörper vorgesehen. Die Zulauföffnungen sind besonders bevorzugt über den gesamten Umfang der Rohrwand verteilt angeordnet, und generieren bei einfließender Lösungsflüssigkeit über den gesamten Umfang eine Vielzahl von Wasserstrahlen, die radial in den Mischraum gerichtet sind.

Grundsätzlich kann das Trockenkonzentrat in dem Zulaufabschnitt oder dem Ablaufabschnitt gelagert sein. Besonders bevorzugt ist das Trockenkonzentrat in dem Mischraumbalg gelagert, also in dem Mischraum. Hierdurch wird unter anderem ermöglicht, dass das Trockenkonzentrat durch Kneten des Mischraumbalgs von außen aufgelockert werden kann, so dass der Lösungsvorgang hierdurch verbessert wird. Ferner lässt sich in dem Mischraumbalg eine verhältnismäßig große Menge an Trockenkonzentrat und eine verhältnismäßig lockere Schüttung des Trockenkonzentrats unterbringen.

Gemäß einer bevorzugten Ausgestaltung der Erfindung ist in dem Rohrkörper stromabwärts der Ablauföffnung ein Auslassfilter angeordnet. Der Auslassfilter ist also fluidisch zwischen der Ablauföffnung und dem Kartuschen- Auslass angeordnet. Der Auslassfilter sorgt dafür, dass kopuskuläre Bestandteile der abfließenden Dialyseflüssigkeit in der Kartusche zurückgehalten werden, beispielsweise ungelöstes Trockenkonzentrat.

Besonders bevorzugt ist in dem Rohrkörper zwischen dem Kartuschen-Einlass und der Zulauföffnung ein Einlassfilter angeordnet, durch den kopuskuläre Bestandteile in der einfließenden Lösungsflüssigkeit zurückgehalten werden. Ferner wird durch den Einlassfilter verhindert, dass bei einer Rückströmung kopuskuläre Bestandteile durch den Kartuschen-Einlass in Gegenrichtung aus der Kartusche ausfließen können.

Vorzugsweise wird der Mischraumbalg von einem elastischen Balgkörper gebildet. Der elastische Balgkörper kann beispielsweise derart konfiguriert sein, dass er sich aufgrund seiner Elastizität nach dem Ende der Nutzung der Kartusche selbsttätig zusammenzieht, beispielsweise auf das Volumen bzw. den Umfang des Rohrkörpers zusammenzieht. Der elastische Balgkörper kann besonders bevorzugt dehnungsbegrenzende Fasern aufweisen, die die maximale Ausdehnung des elastischen Balgkörper begrenzen, um eine Beschädigung des Balgkörpers bei entsprechend hohem Überdruck in dem Mischraum zu vermeiden

Gemäß einer bevorzugten Ausgestaltung der Erfindung ist an dem Einlass-Längsende und an dem Auslass-Längsende des Verbindungskörpers bzw. des Rohrkörpers jeweils eine Kupplungsstruktur angeordnet, wobei die beiden Kupplungsstrukturen verschieden voneinander ausgebildet sind. Die beiden Kupplungsstrukturen sind jeweils annähernd komplementär zu entsprechenden Kupplungsstrukturen einer Kartuschenaufnahme bzw. einer Einspannvorrichtung eines entsprechenden Dialysegerätes ausgebildet. Auf diese Weise wird sichergestellt, dass die Kartusche nur in einer einzigen axialen Orientierung an der Kartuschenaufnahme appliziert werden kann. Hierdurch wird eine hohe Bedienungssicherheit hergestellt.

Im Folgenden wird ein Ausführungsbeispiel der Erfindung anhand der Zeichnungen näher erläutert. Es zeigen:
Figur 1 einen Längsschnitt einer erfindungsgemäßen Dialysetrockenkonzentrat- Kartusche im mit Trockenkonzentrat gefüllten Zustand, und
Figur 2 einen Längsschnitt der Dialysetrockenkonzentrat- Kartusche der Figur 1 im leeren bzw. verbrauchten Zustand.

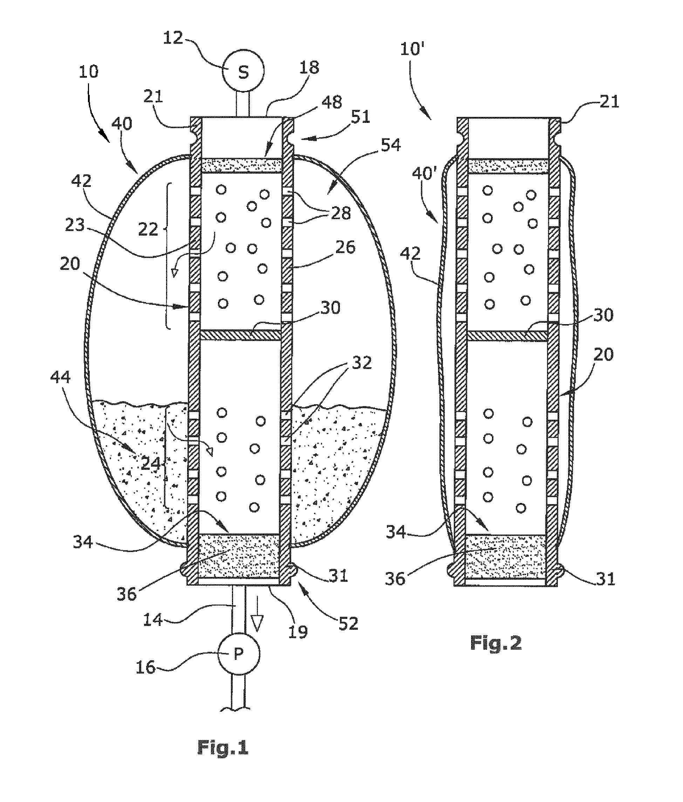

In den Figuren 1 und 2 ist eine Dialysetrockenkonzentrat-Kartusche 10 dargestellt, die konstruktiv abgestimmt ist auf eine entsprechende Kartuschenaufnahme eines Dialysegerätes. Die Kartusche 10 enthält ein in einer Lösungsflüssigkeit lösbares Trockenkonzentrat 44, beispielsweise ein Bicarbonat- Pulver zur Herstellung einer Bicarbonat-Lösung.

In der Figur 1 ist die Kartusche 10 schematisch in einem an ein Dialysegerät in senkrechter Kartuschen-Ausrichtung applizierten Zustand dargestellt. Von einer Flüssigkeitsquelle 12 fließt eine Lösungsflüssigkeit, beispielsweise Dialysewasser, in einen Kartuschen-Einlass 18 durch einen Kartuschen-Mischraum 54 zu einem Kartuschen-Auslass 19, und von Letzterem über eine Dialyseflüssigkeits-Leitung 14 zu einer Dialyseflüssigkeits-Pumpe 16.

Die Kartusche 10 ist im Wesentlichen aus einem Verbindungskörper 20 und einem Mischraumbalg 40 zusammengesetzt. Der Verbindungskörper 20 wird von einem hohlzylindrischen Kunststoff-Rohrkörper 23 gebildet, dessen oberes offenes Längsende 21 den Kartuschen-Einlass 18 und dessen unteres offenes Längsende 31 den Kartuschen Auslass 19 bildet. Ungefähr in der Mitte der axialen Längserstreckung des Rohrkörpers 23 ist eine Trennwand 30 angeordnet, die den oberen auf diese Weise gebildeten Hohlraum innerhalb des Rohrkörpers 23 fluidisch isoliert von dem unteren Hohlraum innerhalb des Rohrkörpers 23.

Im Bereich des oberen Rohrkörper-Hohlraums sind mehrere Zulauföffnungen 28 in Form von Wandbohrungen in der Rohrwand 26 des Rohrkörpers 23 vorgesehen. Durch die Zulaufleitungen 28 wird ein Zulaufabschnitt 22 definiert, in dem die Lösungsflüssigkeit aus dem oberen Rohrkörper-Hohlraum radial nach außen in einen Mischraum 54 austreten kann, der den Verbindungskörper 20 außenseitig ringartig umgibt und durch den Mischraumbalg 40 lateral begrenzt ist.

Der Mischraumbalg 40 wird von einem flexiblen Balgkörper 42 gebildet, der besonders bevorzugt elastisch ausgebildet sein kann, so dass sich der Mischraumbalg 40' bei entleerten Mischraum 54 auf das Außenmaß des Verbindungskörpers 20 zusammenziehen kann, wie in der Figur 2 dargestellt, die die Kartusche 10' im leeren Zustand zeigt.

Im Bereich des unteren Rohrkörper-Hohlraums sind mehrere Ablauföffnungen 32 in Form von Wandbohrungen in der Rohrwand 26. Rohrkörpers 23 vorgesehen. Durch die Ablauföffnungen 32 wird ein Ablaufabschnitt 24 definiert, in dem die Dialyseflüssigkeit, die aus der Lösungsflüssigkeit und dem darin gesättigt gelösten Trockenkonzentrat 44 besteht, radial nach innen aus dem Mischraum 54 in den unteren Rohrkörper-Hohlraum abfließt. Bei Natrium-Bikarbonat-Trockenkonzentrat 44 ist die Sättigung bezogen auf 37°C bei 8,4% erreicht.

In dem Rohrkörper 23 ist fluidisch zwischen dem Kartuschen-Einlass 18 und dem Zulaufabschnitt 22 ein Einlassfilter 48 angeordnet, durch den kopuskuläre Bestandteile in beiden Flussrichtungen zurückgehalten werden. Ferner ist in dem Rohrkörper 23 fluidisch zwischen dem Ablaufabschnitt 24 und dem Kartuschen-Auslass 19 ein Auslassfilter 34 vorgesehen, der aus einem feinporigen Filterkörper 36 besteht. Der Auslassfilter 34 hält ebenfalls kopuskuläre Bestandteile der ausfließenden Flüssigkeit zurück, insbesondere ungelöstes kopuskuläres Trockenkonzentrat.

An dem Einlass-Längsende 21 ist außenseitig in Form einer umlaufenden Ringnut eine Einlass-Kupplungsstruktur 51 vorgesehen. An dem Auslass-Längsende 31 ist außenseitig in Form eines umlaufenden Ringstegs eine Auslass-Kupplungsstruktur vorgesehen. Die beiden Kupplungsstrukturen 51,52 unterscheiden sich derart voneinander, dass die Kartusche 10 ausschließlich in der in den Figuren 1 und 2 dargestellten vertikalen Ausrichtung und Orientierung mit den korrespondierenden Kupplungselementen einer Kartuschenaufnahme des Dialysegerätes fluidisch verbunden werden kann.

## Patentansprüche

1. Dialysetrockenkonzentrat-Kartusche (10) mit einem in einer Lösungsflüssigkeit lösbaren Trockenkonzentrat (44) zur Herstellung einer Dialyseflüssigkeit durch Mischen des Trockenkonzentrats (44) mit einer Lösungsflüssigkeit, mit
einem steifen Verbindungskörper (20) mit einem Kartuschen-Einlass (18) an einem Längsende (21) und einem koaxialen Kartuschen-Auslass (19) an dem anderen Längsende (31),
mindestens einer Zulauföffnung (28) an dem Verbindungskörper (20), durch die die Lösungsflüssigkeit in einen Mischraum (54) fließt, der den Verbindungskörper (20) teilweise umgibt, und mindestens einer Ablauföffnung (32) an dem Verbindungskörper (20), durch den die Lösungsflüssigkeit aus dem Mischraum (54) zu dem Auslass (19) fließt, und
einem separaten flexiblen Mischraumbalg (40), der den Mischraum (54) zwischen der Zulauföffnung (28) und der Ablauföffnung (32) fluidisch abschließt.

2. Dialysetrockenkonzentrat-Kartusche (10) nach Anspruch 1, wobei der steife Verbindungskörper (20) von einem Rohrkörper (23) gebildet ist.

3. Dialysetrockenkonzentrat-Kartusche (10) nach Anspruch 2, wobei in dem Rohrkörper (23) zwischen dem Einlass (18) und dem Auslass (19) eine Trennwand (30) angeordnet ist.

4. Dialysetrockenkonzentrat-Kartusche (10) nach Anspruch 3, wobei in dem Rohrkörper (23) stromaufwärts der Trennwand (30) mehrere Zulauföffnungen (28) und stromabwärts der Trennwand (30) mehrere Ablauföffnungen (30) vorgesehen sind.

5. Dialysetrockenkonzentrat-Kartusche (10) nach einem der vorangegangenen Ansprüche, wobei das Trockenkonzentrat (44) in dem Mischraumbalg (40) gelagert ist.

6. Dialysetrockenkonzentrat-Kartusche (10) nach einem der vorangegangenen Ansprüche 2-5, wobei in dem Rohrkörper (23) stromabwärts der Ablauföffnung (32) ein Auslassfilter (34) angeordnet ist.

7. Dialysetrockenkonzentrat-Kartusche (10) nach einem der vorangegangenen Ansprüche, wobei der Mischraumbalg (40) von einem elastischen Balgkörper (42) gebildet ist.

8. Dialysetrockenkonzentrat-Kartusche (10) nach einem der vorangegangenen Ansprüche 2-7, wobei in dem Rohrkörper (23) zwischen dem Einlass (18) und der Zulauföffnung (28) ein Einlassfilter (48) angeordnet ist.

9. Dialysetrockenkonzentrat-Kartusche (10) nach einem der vorangegangenen Ansprüche 2-8, wobei an dem Einlass- Längsende (21) und an dem Auslass- Längsende (31) jeweils eine Kupplungsstruktur (51,52) angeordnet ist, wobei die beiden Kupplungsstrukturen (51,52) verschieden voneinander ausgebildet sind.
